(19) Europäisches Patentamt
European Patent Office
Office européen des brevets

(11) Numéro de publication : **0 491 593 A1**

(12) **DEMANDE DE BREVET EUROPEEN**

(21) Numéro de dépôt : **91403287.5**

(51) Int. Cl.[5] : **C07D 307/20**

(22) Date de dépôt : **05.12.91**

(30) Priorité : **17.12.90 FR 9015753**

(43) Date de publication de la demande : **24.06.92 Bulletin 92/26**

(84) Etats contractants désignés : **AT BE CH DE DK ES FR GB GR IT LI LU NL SE**

(71) Demandeur : **BEGHIN-SAY SOCIETE ANONYME**
**F-59239 Thumeries (FR)**

(72) Inventeur : **Defaye, Jacques**
**202, chemin du Vercors**
**F-38330 Saint Dismier (FR)**
Inventeur : **Gadelle, Andrée**
**23, Hameau Fleuri, Mont Bonnot**
**F-38330 Saint Dismier (FR)**
Inventeur : **Pedersen, Christian**
**Bredesvinget 18**
**DK-2830 Virum (DK)**

(74) Mandataire : **Dorland, Anne-Marie et al**
**BEGHIN-SAY Service Propriété Industrielle 23 boulevard Georges Clemenceau**
**F-92402 Courbevoie Cédex (FR)**

(54) **Anhydrides d'alditylamines et procédé pour les préparer.**

(57) Nouveaux 1-amino-1-désoxy-3,6-anhydro-hexitols **1** et 1-amino-1-désoxy-2,5-anhydro-pentitols **2** de formule générale (I).

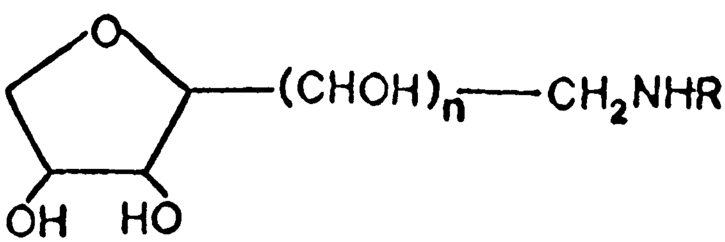

(I)

où n = 1 pour les composés **1** et n = 0 pour les composés **2** et dans laquelle R est soit H soit $COR^1$.

Procédé de préparation des nouveaux anhydrides d'alditylamines de formule (I) caractérisé par le fait que l'on met en réaction un 1-amino-1-désoxy-alditol (glycamine) avec au moins un acide carboxylique, un anhydride ou un halogénure d'acide correspondant dans du fluorure d'hydrogène liquide à une température inférieure à environ 50°C, cette réaction étant le cas échéant suivie d'une transformation des anhydro-glycamines obtenues en amides correspondantes au moyen d'un acide carboxylique, d'une façon connue en soi.

EP 0 491 593 A1

La présente invention se rapporte à de nouveaux anhydrides d'alditylamines ainsi qu'à des procédés pour les préparer. Plus précisément, l'invention concerne de nouveaux 1-amino-1-désoxy-3,6-anhydro-hexitols (3,6-anhydro-hexitylamines) et 1-amino-1-désoxy-2,5-anhydro-pentitols (2,5-anhydro-pentitylamines).

De tels composés peuvent trouver de nombreuses applications, notamment dans le domaine des surfactants ou des polymères, où le fait que leur groupement aminé peut être fonctionnalisé les rend particulièrement intéressants, ainsi qu'en pharmacie.

La littérature antérieure a décrit des composés voisins, les 2,5-diamino-1,4-3,6-dianhydro-mannitol et sorbitol (R. Montgomery et L.F. Wiggins, J. Chem. Soc. (1946), 393) et le 5-amino-5-désoxy-1-4-3,6-dianhydro-L-iditol 2-nitrate (K. Klessing, S.S. Chatterjee, demande de brevet européen EP 44940) qui ont été préparés en plusieurs étapes partant des 1,4-3,6-dianhydro-mannitol et sorbitol.

Les nouveaux 1-amino-1-désoxy-3,6-anhydro-hexitols **1** et 1-amino-1-désoxy-2,5-anhydro-pentitols **2**, objets de la présente invention, peuvent être représentés par la formule générale (I) ci-dessous :

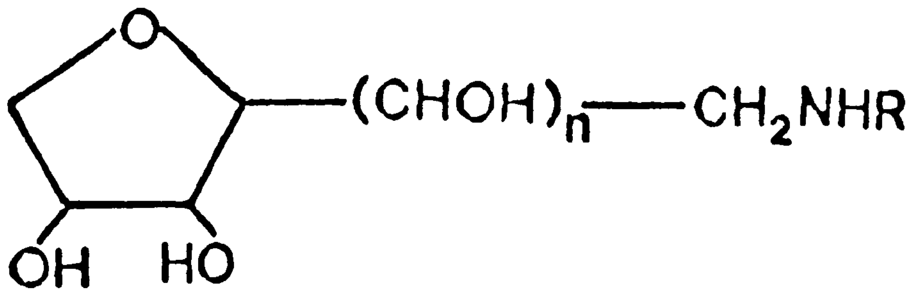

(I)

où n= 1 pour les composés **1**
et n = 0 pour les composés **2**
et dans laquelle le radical R est soit H soit $COR^1$, avec $n \neq 0$ si R = H.

Le radical $R^1$ est constitué par un atome d'hydrogène ou par un radical hydrocarboné saturé ou insaturé, linéaire ou ramifié, substitué ou non substitué, ayant par exemple de 1 à 20 atomes de carbone, de préférence de 1 à 15 atomes de carbone. A titre d'exemple de tels radicaux hydrocarbonés, on peut citer le radical méthyle. $R^1$ peut être également un radical cycloalkyle, polycycloalkyle ou encore aromatique.

Dans le brevet WO 8900162 de la demanderesse, on a décrit un procédé amélioré pour la préparation de 1,4-3,6-dianhydro-hexitols par déshydratation d'hexitols dans le fluorure d'hydrogène en présence d'un acide carboxylique, de l'anhydride d'acide carboxylique ou encore de l'halogénure d'acide correspondant. On a également appliqué ce procédé à la préparation d'hexonolactones, de 1,4- ou 3,6-monoanhydro-hexitols, de 3,6-anhydro-hexoses ainsi que d'alkyl-3,6-anhydro-hexofuranosides.

Les nouveaux composés **1** et **2** de la présente invention peuvent être obtenus comme dans le brevet WO 8900162 précité, en utilisant le fluorure d'hydrogène à l'état liquide, en présence d'au moins un acide carboxylique, ou d'un anhydride ou d'un halogénure d'acide correspondant.

Dans une variante du procédé qui permet de préparer les 1-amino-1-désoxy-3,6-anhydro-hexitols **1** et 1-amino-1-désoxy-2,5-anhydro-pentitols **2**, on a montré que l'acide sulfurique M à 10 M, à une température comprise entre environ 80 et 140°C, particulièrement l'acide sulfurique environ 6 M sensiblement à ébullition (≈ 120°) pouvait néanmoins être utilisé. Les rendements de la réaction sont toutefois, et dans tous les cas, inférieurs.

Les 1-amino-1-désoxy-3,6-anhydro-hexitols **1** et les 1-amino-1-désoxy-2,5-anhydro-pentitols **2** sont préparés selon la présente invention par réaction de la glycamine (1-amino-1-désoxy-alditol) correspondante avec au moins un acide carboxylique ou un anhydride ou un halogénure d'acide correspondant dans du fluorure d'hydrogène liquide à une température inférieure à environ 50°C.

Les 1-amino-1-désoxy-alditols de départ sont commodément préparés dans la littérature par réduction catalytique d'un aldose en présente d'hydrazine (R.U. Lemieux, brevet US 2,830,983) ou par réduction catalytique d'une benzylglycamine (F. Kagan, M.A. Rebenstorf et E.J. Heinzelmann, J. Am. Chem. Soc. 79 (1957) 3541). On pourra utiliser indifféremment comme produits de départ les glycamines ou leurs sels, chlorhydrates, bromhydrates, etc...

Pour les composés **1** et **2** dans lesquels le radical R est $COR^1$, on fera réagir un acide carboxylique sur les anhydro-glycamines obtenues pour les transformer en amides correspondantes de façon connue en soi.

Pour des raisons de simplification de la terminologie, on sera amené à utiliser, dans la présente description, le terme "acide carboxylique" pour désigner aussi bien l'acide carboxylique que l'anhydride ou l'halogénure d'acide correspondant. De la même façon, l'expression "acide carboxylique" pourra représenter, le cas

échéant, plusieurs acides carboxyliques.

L'acide formique est l'acide carboxylique le plus couramment utilisé en raison de son faible coût, ainsi que de sa facilité d'élimination en fin de réaction. Des homologues supérieurs peuvent être néanmoins utilisés, de même que les anhydrides d'acides carboxyliques ou les anhydrides d'acides correspondants mais, dans ce cas, la réaction est plus lente.

L'action de l'acide carboxylique étant catalytique, il n'est pas nécessaire d'en utiliser des proportions stoechiométriques par rapport au composé de départ. Ainsi, le rapport molaire de l'acide carboxylique au composé de départ pourra être compris entre 0,1 et 1, sans qu'il soit exclu qu'un rapport plus faible ou plus élevé donne aussi des résultats. On a remarqué cependant que, lorsque la proportion d'acide carboxylique dépassait le seuil de l'équimolarité, ceci avait pour effet, en diluant le fluorure d'hydrogène, de diminuer corrélativement la vitesse de déshydratation du produit de départ mis en réaction et d'augmenter le temps d'obtention des anhydrides de ce produit. En effet, la concentration optimale de la glycamine de départ par rapport au fluorure d'hydrogène se situe dans un rapport 1/4. Toutefois, il doit être entendu que, dans la pratique, la concentration peut varier autour de cette valeur optimale.

La réaction est généralement réalisée à température ambiante, température à laquelle le fluorure d'hydrogène qui a dissous les produits de départ est liquide. Cependant, une légère élévation de la température, à 40° ou 50°C, peut permettre de réduire le temps de réaction .Dans ce cas, un récipient clos doit être utilisé de façon à ce que le fluorure d'hydrogène reste sous forme liquide. De même, une température inférieure à la température ambiante peut être choisie, par exemple 0°C.

En fin de réaction, les réactifs sont éliminés par entraînement par un courant d'air ou de gaz inerte, opération suivie éventuellement d'un entraînement par un cosolvant. Les résidus acides peuvent, lorsque cela est nécessaire, être neutralisés par action d'agents alcalins ou passage de la solution aqueuse sur résine échangeuse d'ions.

Une mise en oeuvre préférée du procédé de l'invention est décrite ci-dessous. Dans un récipient en polyéthylène, ou encore en acier, on additionne la glycamine d'environ quatre parties de fluorure d'hydrogène et d'une proportion équimoléculaire par rapport à la glycamine, d'un acide carboxylique, de son anhydride, ou de l'halogénure d'acide correspondant. Le mélange réactionnel est alors maintenu à température voisine de l'ambiante pendant des durées de l'ordre de 24 h. Après élimination des réactifs, ou les anhydro-glycamines **1**, **2** sont obtenues sous forme amorphe et le plus souvent recristallisées dans un solvant adéquat.

Les exemples qui sont donnés porteront sur la préparation des 1-amino-1-désoxy-3,6-anhydro-D-glucitol **3**, 1-amino-1-désoxy-3,6-anhydro-D-mannitol **4** et 1-amino-1-désoxy-2,5-anhydro-D-xylitol **5** puisque les précurseurs glycamine correspondants sont les plus accessibles. Il va de soi cependant que le procédé s'applique à toute hexitylamine et pentitylamine. Les anhydro-glycitylamines **3-5** peuvent ensuite être commodément transformées en les amides correspondantes par action d'acides carboxyliques selon des procédés connus, ou encore conservées sous forme de sels, comme cela est décrit dans les Exemples.

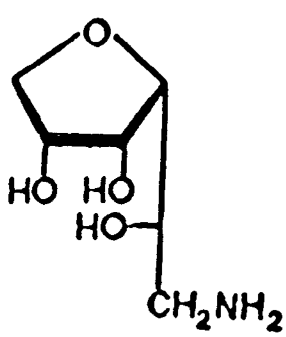
3

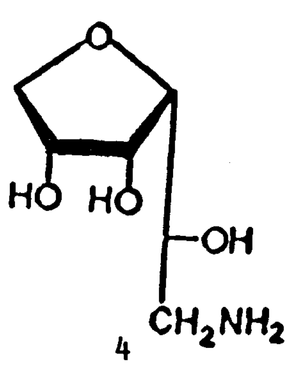
4

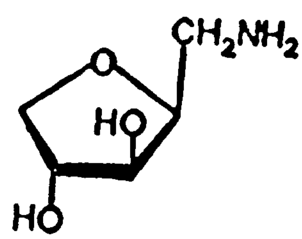
5

Les Exemples suivants illustrent l'invention sans la limiter.

Exemple 1 : Préparation du 1-amino-1-désoxy-3,6-anhydro-D-glucitol fluorhydrate.

Le 1-amino-1-désoxy-D-glucitol (1 g) est dissous dans le fluorure d'hydrogène (5 ml) et additionné d'acide formique (0,2 ml). La solution est maintenue à 20°C pendant 24 h puis concentrée par passage d'un courant d'air ou encore sous pression réduite conduisant à une huile (1,2 g, rendement sensiblement quantitatif) caractérisée sous la forme de son dérivé tri-O-acétyl-1-désoxy-1-phtalimido obtenu par action successive de l'anhydride phtalique et de l'anhydride acétique dans la pyridine p.f. 145-146°C, $[\alpha]_D$ + 31° (c 0,1, chloroforme).

Exemple 2 : Préparation du 1-amino-1-désoxy-3,6-anhydro-D-mannitol.

a) Dans le fluorure d'hydrogène :

Le 1-amino-1-désoxy-D-mannitol (1,2 g) dans le fluorure d'hydrogène (5 ml) est additionné d'acide formique (0,2 ml) et la solution est maintenue à température ambiante pendant 24 h puis concentrée sous pression réduite. Le résidu est redissous à deux reprises dans le méthanol et la solution est concentrée. Le résidu, constitué par le fluorhydrate de 1-amino-1-désoxy-3,6-anhydro-D-mannitol, cristallise dans l'éthanol (880 mg, 80 %), p.f. 166-168°C, $[\alpha]_D$-8,5° (c 1,2, eau).

b) Dans l'acide sulfurique :

Le 1-amino-1-désoxy-D-mannitol (1,5 g) est dissous dans l'acide sulfurique 6 M (5 ml) et la solution est portée à reflux pendant 4 jours. Le mélange réactionnel est alors neutralisé par passage sur une colonne de résine Amberlite IRA-400 ($OH^-$, 20ml) puis la solution est acidifiée par addition d'acide chlorhydrique. La solution est concentrée sous pression réduite et le résidu, constitué par le 1-amino-1-désoxy-3,6-anhydro-D-mannitol sous forme chlorhydrate, cristallise dans l'éthanol (690 mg, 50 %), p.f. 165-167°C, $[\alpha]_D$-8,5°(c 0,09, eau).

Exemple 3 : Préparation du 1-amino-1-désoxy-2,5-anhydro-D-xylitol fluorhydrate.

Le bromhydrate de 1-amino-1-désoxy-D-xylitol (1,5 g) est additionné de fluorure d'hydrogène (4 ml) et d'acide formique (0,2 ml) et la solution est maintenue à température ambiante pendant 24 h, puis concentrée par passage d'un courant d'air ou sous pression réduite. Le résidu est ensuite coévaporé avec du méthanol (10 ml) à deux reprises et recristallisé dans l'eau chaude (0,5 ml) par addition d'éthanol (10 ml) (530 mg, 81 %), p.f. 179-181°C, $[\alpha]_D$+0.8° (c 0,01, eau).

## Revendications

1. Nouveaux anhydrides d'alditylamines de formule générale (I) ci-dessous :

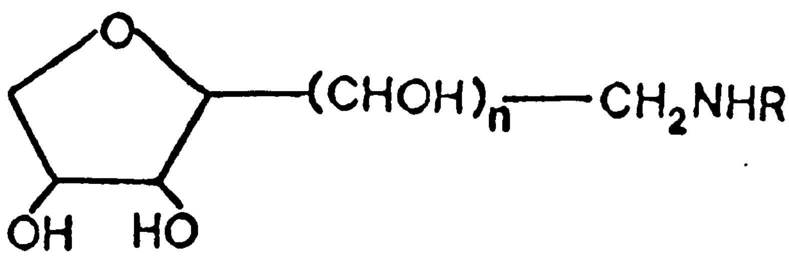

(I)

dans laquelle n est un nombre entier égal à 0 ou 1 et R est choisi parmi H et les radicaux de formule $COR^1$, où $R^1$ est soit H soit un radical hydrocarboné $R^2$, avec $n \neq 0$ si R = H

2. Nouveaux 1-amino-1-désoxy-3,6-anhydro-hexitols selon la revendication 1 caractérisés par le fait que le nombre entier n de la formule (I) est égal à 1.

3. Nouveaux 1-amino-1-désoxy-2,5-anhydro-pentitols selon la revendication 1 caractérisés par le fait que le nombre entier n de la formule (I) est égal à 0.

4. Composés selon l'une quelconque des revendications 1 à 3 dans lesquels ledit radical hydrocarboné $R^2$ a de 1 à 20 atomes de carbone, en particulier de 1 à 15 atomes de carbone, et est choisi parmi les radicaux alkyle, cycloalkyle, polycycloalkyle et aryle.

5. Composés selon la revendication 4 dans lesquels ledit radical hydrocarboné $R^2$ est le radical méthyle.

6. Procédé de préparation des anhydrides d'alditylamines selon l'une quelconque des revendications 1 à 5 dans lequel on met en réaction un 1-amino-1-désoxy-alditol (glycamine) avec au moins un acide carboxylique, un anhydride ou un halogénure d'acide correspondant dans du fluorure d'hydrogène liquide à une température inférieure à environ 50°C, cette réaction étant le cas échéant suivie d'une transformation des anhydro-glycamines obtenues en amides correspondantes au moyen d'un acide carboxylique, d'une façon connue en soi.

7. Procédé selon la revendication 6 dans lequel ledit acide carboxylique de départ est l'acide formique.

8. Procédé selon l'une quelconque des revendications 6 et 7 dans lequel le rapport molaire dudit acide carboxylique ou anhydride ou halogénure d'acide par rapport à la glycamine de départ est compris entre 0,1 et 1.

9. Procédé de préparation des alditylamines selon l'une quelconque des revendications 1-5 dans lequel on met en réaction un 1-amino-1-désoxy-alditol dans de l'acide sulfurique environ M à environ 10 M, à une température d'environ 80 à 140 °C et on neutralise le mélange réactionnel formé en fin de réaction.

Office européen des brevets

# RAPPORT DE RECHERCHE EUROPEENNE

Numero de la demande

EP 91 40 3287

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X | BULLETIN DE LA SOCIETE CHIMIQUE DE FRANCE. vol. 67, 1967, PARIS FR pages 104 - 107; J. CLEOPHAX ET AL.: 'Analogs non glycosidiques de nucléosides. I. - (Anhydro-2',5'-D-ribitylamino)-6-purine' * page 105, colonne de gauche, exemple 5; page 104, colonne de gauche, lignes 1-6 * | 1,3 | C07D307/20 |
| Y | US-A-2 891 052 (F.E. BOETTNER) * colonne 2, ligne 60 - ligne 65; revendication 1 * | 1,2,6,7 | |
| D,Y | WO-A-8 900 162 (BEGHIN-SAY S.A.) * abrégé; revendications 1,2 * | 1,2,6,7 | |
| D,Y | US-A-2 830 983 (R.U. LEMIEUX) * colonne 2; revendication 1; exemples 1-7 * | 1,2,6,7 | |
| A | IL FARMACO, ED. SC. vol. 34, no. 7, 1979, pages 635 - 645; V. CAVRINI ET AL: 'Homoanalogues des nucléosides pyrimidiques. XII - 3,6-Anhydro-1,2-bis-désoxy-1-(pyrimidin- et 5-halopyrimidin-1-yl)-D-arabino-hexitols; 3,6-anhydro-1-désoxy-1-(5-halopyrimidin-1-yl)-D-glucitols' * pages 644, 645, exemples XV, XVI; page 639, paragraphe B * | 1,2 | DOMAINES TECHNIQUES RECHERCHES (Int. Cl.5 ) C07D |

Le présent rapport a été établi pour toutes les revendications

| Lieu de la recherche | Date d'achèvement de la recherche | Examinateur |
|---|---|---|
| LA HAYE | 26 MARS 1992 | PAISDOR B. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)